# EUROPEAN PATENT APPLICATION

(11) **EP 4 760 242 A1**
(43) Date of publication of application: **17.06.2026**
(21) Application number: 23951524.0
(22) Date of filing: 07.09.2023
(51) Int. Cl.: G01N 21/898

(54) **SINGLE PLATE DEFECT DETERMINATION DEVICE, DEFECT DETERMINATION METHOD, AND DEFECT DETERMINATION PROGRAM**

(71) Applicant: Meinan Machinery Works, Inc., Obu-shi, Aichi 474-8543 (JP)
(72) Inventor: MORITA, Koji, Obu-shi, Aichi 474-8543 (JP); HAMAJIMA, Ryota, Obu-shi, Aichi 474-8543 (JP)
(74) Representative: Vossius & Partner Patentanwälte Rechtsanwälte mbB
(86) International application number: PCT/JP2023/032673
(87) International publication number: WO 2025/052626

(57) **Abstract**

Holes H1, H2, and H3 are detected in a continuous veneer 101B; the length of the veneer formed through peeling by one rotation in each round of a log is calculated as veneer lengths L1, L2, and L3 per round; the shapes of the detected holes obtained in specific rounds are aligned with positions after one rotation that are shifted to an upstream side in a transfer direction by the veneer lengths per round, respectively; and it is estimated that insufficient-thickness portions T2, T3, and T4, which are formed as a recessed portion having an inclination on a surface layer portion of the log is cut, may be included in regions defined by the aligned shapes of the holes obtained in the specific rounds. Accordingly, it is possible to estimate regions that may include the insufficient-thickness portions T2, T3, and T4, which cannot be easily determined by a method of observing a difference in color of reflected light or a captured image.

## Description

### Technical Field

The present invention relates to a veneer defect determination device, a defect determination method, and a defect determination program, and is more particularly suitable for a system that estimates an insufficient-thickness defect present in a veneer cut from a log.

### Background Art

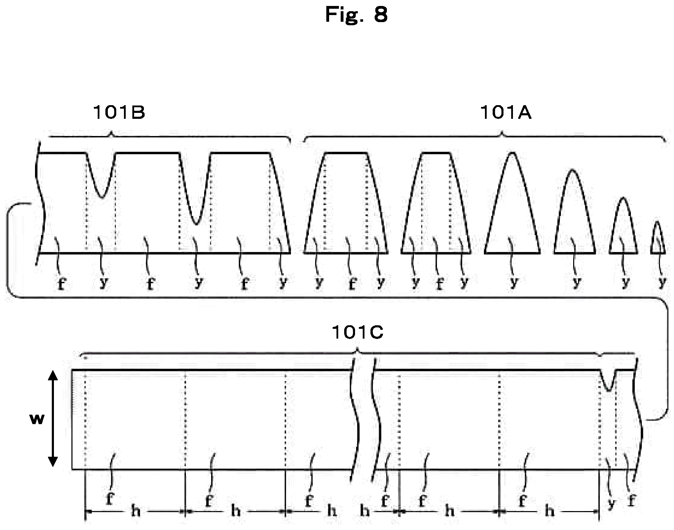

Generally, veneer sheets (hereinafter simply referred to as "veneers") used to manufacture boards such as plywood and laminated veneer lumber are produced by cutting logs, which are natural objects, using a veneer lathe. When a veneer is formed through peeling from a log by a veneer lathe, as shown in FIG. 8, a discontinuous veneer 101A including only unusable portions y that cannot be used to manufacture boards or including usable portions f, which can be used to manufacture boards, in addition to unusable portions y, and a connected continuous veneer 101B including unusable portions y and usable portions f are formed through peeling from the irregular outer peripheral portion of the log. Further, after the log is cut into a substantially columnar shape, a continuous veneer 101C including only usable portions f without any unusable portions y is formed through peeling. The usable portion f refers to a portion from which a rectangular region having a required width w necessary for boards can be ensured.

When each of the veneers 101A to 101C is used to manufacture plywood, laminated veneer lumber, or the like, corresponding forming processing is required. For example, unusable portions y need to be removed from the veneers 101A and 101B. More preferably, in addition to removing unusable portions y, it is even more appropriate to connect the usable portions f to form a veneer joined to required dimensions. On the other hand, the veneer 101C including only usable portions f may be simply sequentially cut into a predetermined length h so that veneers having required dimensions (hereinafter referred to as predetermined-length veneers) is sequentially formed.

The following technique is known (see, for example, PTL 1). In the technique, the total length of a strip-shaped veneer formed through peeling in a case where a log is cut down to a prescribed peeling core diameter is predictively calculated, a specific point is identified at which a back-calculated distance from a boundary position between a veneer including the above-described unusable portions and a veneer including no unusable portion to the peeling core of the log is within the range of the predictively calculated total length and is an integer multiple of the predetermined length of the predetermined-length veneer, and predetermined-length cutting processing is started using the specific point as a starting position for predetermined-length cutting.

In the related art including the invention disclosed in PTL 1, the detection of the unusable portions of a veneer formed through peeling by a veneer lathe is performed by veneer detection mechanisms installed at a plurality of positions in a direction orthogonal to a veneer transport direction. Here, since a concave portion present on the outer peripheral portion of a log appears as a hole in a veneer to be formed through peeling, a portion where the veneer is not present is detected as a hole and a veneer portion included in a rectangular region having a required veneer width, which is set so that the detected hole is inscribed in the rectangular region, is detected as the unusable portion.

However, a log, which is a natural object, does not always include recesses perpendicular to a normal direction of the log, that is, the thickness direction of the veneer, but instead forms recesses having inclinations. Since a knife of a veneer lathe performs cutting along a path set such that the outer peripheral portion of the log is peeled off at a constant thickness, the veneer formed through peeling from the surface layer of the log includes an inclined portion that gradually decreases in thickness toward a hole portion. That is, in most cases, an inclined portion having an insufficient thickness is present around the hole portion. Although it is desirable that this insufficient-thickness portion also be detected as an unusable portion, this is not mentioned in PTL 1.

Here, in a case where a photoelectric sensor or the like for detecting the presence or absence of an object using reflected light from the object is used as the veneer detection mechanism, it is possible to detect the presence or absence of the veneer (the presence or absence of a hole portion). However, around the hole portion, it is not possible to detect an insufficient-thickness portion since the photoelectric sensor can merely determine that the veneer is present.

Further, a defect inspection device (see, for example, PTL 2) is known that captures an image of a veneer using imaging means while irradiating the veneer with visible light, and detects a defective portion caused by discoloration of the veneer using the color distribution of the captured image. However, merely observing a color difference (a difference in color) of an image captured under irradiation with visible light makes it difficult to determine an insufficient-thickness portion in which a difference in hue from a normal portion is unlikely to occur.

### Citation List

### Patent Literature

PTL 1: JP2009-160834A
PTL 2: JP2007-147442A

### Summary of Invention

### Technical Problem

The present invention has been made to solve these problems, and an object of the present invention is to make it possible to estimate a region that may include an insufficient-thickness portion which cannot be easily determined by a method of determining whether or not an object is present with reflected light using a photoelectric sensor or the like and a method of observing a difference in color of a captured image.

### Solution to Problem

In order to solve the above-described problems, in the present invention, a hole is detected in a continuous veneer formed through peeling from a log rotating about a spindle center by a veneer lathe and transferred on a transfer path, and a length of the veneer formed through peeling by one rotation is calculated as a veneer length per round in each round of the log. Then, a shape of a hole, which is obtained in a specific round and detected, is aligned with a position after one rotation that is shifted to an upstream side in a transfer direction by the veneer length per round, and it is estimated that an insufficient-thickness portion may be included in a region defined by the aligned shape of the hole obtained in the specific round.

### Advantageous Effects of Invention

According to the present invention configured as described above, it can be estimated that an insufficient-thickness portion, which is formed as a recessed portion having an inclination on a surface layer portion of a log is cut by a knife of a veneer lathe, may be included in a region with which the shape of a hole detected is aligned based on a veneer length per round in each round in each round of the log. That is, according to the present invention, it is possible to estimate a region that may include the insufficient-thickness portion, which is included in the surface layer portion of the log and cannot be easily determined by a method of determining whether or not an object is present with reflected light using a photoelectric sensor or the like and a method of observing a difference in color of a captured image.

### Brief Description of Drawings

[FIG. 1] FIG. 1 is a diagram showing a schematic configuration example of a processing line for logs to which a veneer defect determination device according to the present embodiment is applied.
[FIG. 2] FIG. 2 is a block diagram showing a functional configuration example of the defect determination device according to the present embodiment.
[FIG. 3] FIG. 3 is a diagram showing examples of holes detected by a hole detection unit of the present embodiment.
[FIG. 4] FIG. 4 is a schematic diagram illustrating the processing contents of an insufficient-thickness estimation unit of the present embodiment.
[FIG. 5] FIG. 5 is a flowchart showing an operation example of the defect determination device according to the present embodiment.
[FIG. 6] FIG. 6 is a diagram showing an example in which concave portions are present at a plurality of positions on a surface layer portion of a log.
[FIG. 7] FIG. 7 is a block diagram showing another functional configuration example of the defect determination device according to the present embodiment.
[FIG. 8] FIG. 8 is a diagram showing an example of a veneer that is formed through peeling from a log by a veneer lathe.

### Description of Embodiments

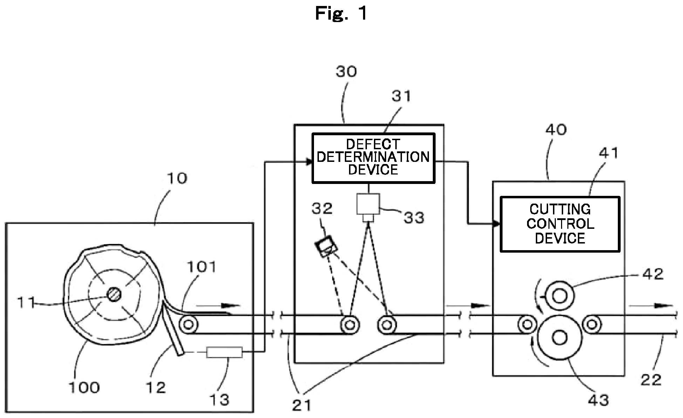

Hereinafter, an embodiment of the present invention will be described with reference to the drawings. FIG. 1 is a diagram showing a schematic configuration example of a processing line for logs to which a veneer defect determination device according to the present embodiment (hereinafter simply referred to as a defect determination device) is applied. As shown in FIG. 1, the processing line to which the defect determination device according to the present embodiment is applied includes a veneer lathe 10, veneer transfer conveyors 21 and 22, a defect inspection system 30, and a veneer cutting apparatus 40.

In the processing line shown in FIG. 1, a log 100 rotating about a spindle center 11 is cut by a knife 12 of the veneer lathe 10 to form a veneer 101 through peeling, and the veneer 101 is transferred by the veneer transfer conveyors 21 and 22 and is sequentially cut into a predetermined length by the veneer cutting apparatus 40. A defect is determined by the defect inspection system 30 while the veneer 101 is transferred to the veneer cutting apparatus 40 from the veneer lathe 10, and a position at which the veneer 101 is cut by the veneer cutting apparatus 40 is controlled based on determination results of the defect inspection system 30.

The knife 12 of the veneer lathe 10 is gradually translated in a direction of the spindle center 11 during rotation of the log 100, and cuts a surface of the log 100 as in so-called "rotary peeling" to form the veneer 101 through peeling. That is, in the veneer lathe 10, both end faces of the log 100 are rotatably supported by spindles each of which includes a jaw portion (chuck) at a distal end thereof, and the knife 12 is moved at a predetermined speed toward the spindle center 11, which is the center of rotation of the log 100, during the rotation of the log 100 at a constant speed, so that the veneer 101 having a constant thickness d is formed through peeling.

A horizontal movement distance of the knife 12 per rotation of the log 100 is the thickness d of the veneer 101 to be formed through peeling, and this value is set in advance. The veneer lathe 10 is provided with a knife movement distance sensor 13, and is configured such that the horizontal movement distance of the knife 12 is detected. The defect inspection system 30 is notified of the horizontal movement distance of the knife 12 detected by the knife movement distance sensor 13.

The defect inspection system 30 includes a defect determination device 31, an illumination device 32, and a line sensor camera 33, and detects or estimates defects present in the veneer 101 transferred by the veneer transfer conveyors 21. In the present embodiment, the defects detected by the defect inspection system 30 are holes (holes penetrating the veneer 101 from the surface side of the veneer 101 to the back side thereof). The holes mentioned here include holes that are formed as a recess having an inclination is cut by the knife 12 of the veneer lathe 10, that is, holes that are present at a tip where the thickness has gradually decreased. Further, the defects estimated by the defect inspection system 30 are regions in which insufficient-thickness portions may be included (hereinafter referred to as potential insufficient-thickness regions). Note that the defect inspection system 30 can also detect other defects; however, the description thereof will be omitted since such defects are not the subject matter of the present invention.

As shown in FIG. 8, when the veneer 101 is formed through peeling from the log 100 by the veneer lathe 10, each of a discontinuous veneer 101A and a continuous veneer 101B, which includes unusable portions y in addition to usable portions f, is formed through peeling from an irregular outer peripheral portion of the log 100. Further, after the log 100 is cut into a substantially columnar shape, a continuous veneer 101C including only usable portions f is formed through peeling. The estimation of the potential insufficient-thickness region is performed for the continuous veneer 101B.

In the related art shown in FIG. 8, a veneer portion included in a rectangular region having a required veneer width w, which is set such that a detected hole is inscribed, is detected as the unusable portion y. In contrast, in the present embodiment, an unusable portion also including a potential insufficient-thickness region is detected in a case where the potential insufficient-thickness region is estimated. For example, a veneer portion included in a rectangular region having a required veneer width w, which is set such that the potential insufficient-thickness region is inscribed, is detected as an unusable portion. Note that the detection of the unusable portion may be performed in the defect determination device 31 or may be performed in a cutting control device 41 of the veneer cutting apparatus 40.

The illumination device 32 irradiates the surface of the veneer 101, which is transferred by the veneer transfer conveyors 21, with visible light. For example, a white light source, such as LEDs, is used as a light source of the visible light. The illumination device 32 linearly extends in a direction (a width direction of the veneer 101) orthogonal to a transfer direction of the veneer 101, and irradiates the veneer 101 with white visible light in a strip shape. Note that an example in which white visible light is used has been described here, but the present invention is not limited thereto. For example, blue visible light, green visible light, or the like may be used.

The line sensor camera 33 captures an image of the surface of the veneer 101, which is formed through peeling from the log 100 rotating about the spindle center 11 by the veneer lathe 10 and is transferred by the veneer transfer conveyors 21, in color to generate the image. The line sensor camera 33 linearly extends in a direction (the width direction of the veneer 101) orthogonal to the transfer direction of the veneer 101, and captures an image of the veneer 101 in a line-by-line manner. While the veneer 101 is being transferred on the veneer transfer conveyors 21, the line sensor camera 33 repeatedly generates a single-line image every predetermined sampling time and sequentially outputs the generated images to the defect determination device 31.

The line sensor camera 33 includes a light-receiving element sensitive to the white visible light that is applied by the illumination device 32. The line sensor camera 33 receives reflected light of the white visible light, which is applied by the illumination device 32, from the veneer 101 and photoelectrically converts the received light to generate the single-line captured image of the veneer 101.

The defect determination device 31 detects or estimates defects present in the veneer 101 by analyzing the captured images output from the line sensor camera 33 (in the following description, "detect or estimate" may be collectively referred to as "determine" unless particularly distinguished from each other). As described above, the defects to be determined in the present embodiment are the holes and the potential insufficient-thickness regions. The estimation of the potential insufficient-thickness region is performed using the captured images acquired from the line sensor camera 33 and the horizontal movement distance of the knife 12 acquired from the knife movement distance sensor 13. The veneer cutting apparatus 40 is notified of information on the determination results for defects by the defect determination device 31. The details of the processing of the defect determination device 31 will be described later.

The veneer cutting apparatus 40 includes the cutting control device 41, a cutting blade 42, and a blade receiver 43. The cutting control device 41 controls the operation of the cutting blade 42 based on the determination results for defects determined by the defect determination device 31, and cuts the continuous veneer 101. For example, the veneer cutting apparatus 40 cuts the veneer 101 at positions where unusable portions are removed from the discontinuous veneer 101A and the continuous veneer 101B including the unusable portions (including the potential insufficient-thickness regions) such that rectangular usable portions remain. On the other hand, the continuous veneer 101C including no unusable portion is sequentially cut for each predetermined length h, so that predetermined-length veneers are sequentially formed.

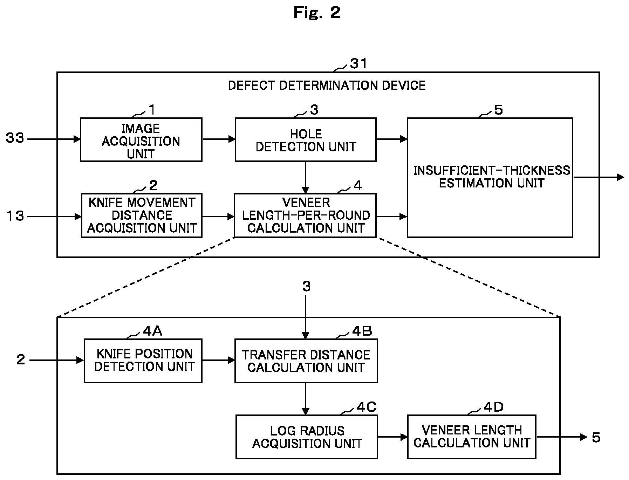

FIG. 2 is a block diagram showing a functional configuration example of the defect determination device 31 according to the present embodiment. As shown in FIG. 2, the defect determination device 31 according to the present embodiment includes an image acquisition unit 1, a knife movement distance acquisition unit 2, a hole detection unit 3, a veneer length-per-round calculation unit 4, and an insufficient-thickness estimation unit 5 as functional components. The veneer length-per-round calculation unit 4 includes a knife position detection unit 4A, a transfer distance calculation unit 4B, a log radius acquisition unit 4C, and a veneer length calculation unit 4D as specific functional components.

These functional blocks 1 to 5 can be formed of any of hardware, digital signal processors (DSPs), and software. For example, the functional blocks 1 to 5 are implemented by the operation of a defect determination program stored in a storage medium, such as a RAM, a ROM, a hard disk, or a semiconductor memory, under the control of a microcomputer including a CPU, a RAM, a ROM, and the like.

The image acquisition unit 1 sequentially acquires single-line captured images that are generated every predetermined sampling time by the line sensor camera 33. Then, the acquired captured images for the respective lines are synthesized (combined), so that the image of the veneer 101 is generated. The generated image of the veneer 101 includes images of the discontinuous veneer 101A, the continuous veneer 101B including unusable portions, and the continuous veneer 101C including no unusable portion. Note that the discontinuous veneer 101A may not be present depending on the shape of the log 100.

The knife movement distance acquisition unit 2 sequentially acquires information on the horizontal movement distance of the knife 12 detected by the knife movement distance sensor 13. Note that, when a hole is detected first from the veneer 101 by the hole detection unit 3, the knife movement distance acquisition unit 2 may transmit an information acquisition request to the knife movement distance sensor 13 and acquire information on the horizontal movement distance of the knife 12 as a response thereto.

The hole detection unit 3 detects holes present in the veneer 101 by analyzing the captured image of the veneer 101 that is acquired by the image acquisition unit 1. As described above, the holes detected by the hole detection unit 3 are holes penetrating the veneer 101 from the surface side of the veneer 101 to the back side thereof perpendicularly (in a thickness direction) (including holes present at a tip where the thickness has gradually decreased). The hole detection unit 3 stores the shapes of the detected holes. The stored shapes of the holes are used for the estimation of the potential insufficient-thickness regions as described later.

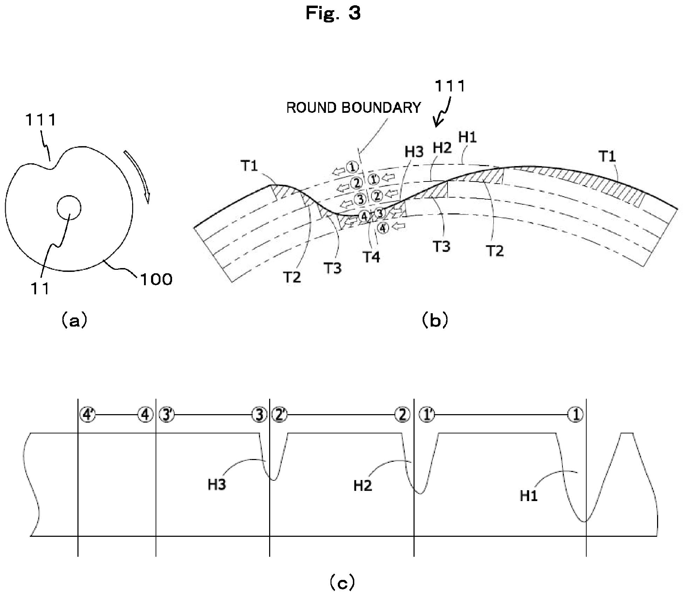

FIG. 3 is a diagram showing examples of the holes detected by the hole detection unit 3. In FIG. 3(a) shows the cross section of the log 100 that is being cut. A concave portion 111 having inclined surfaces is present on a surface layer portion of the log 100. When the log 100 is cut at a constant thickness d while being rotated, the periphery of the concave portion 111 is cut as shown in FIG. 3(b). Further, as shown in FIGs. 3(b) and 3(c), holes H1, H2, and H3 are sequentially detected in each round by the hole detection unit 3. In FIG. 3(c), the right side is the downstream in the transfer direction and the left side is the upstream in the transfer direction.

The large hole H1 is formed on the outermost side by the first round of cutting, and is detected by the hole detection unit 3. The hole H1 detected here is a region in which there is no log 100, that is, a region in which the space of the concave portion 111 is present, on a path shown by a two-dot chain line in FIG. 3(b) and along which the knife 12 passes. As shown by a hatched region in FIG. 3(b), portions not satisfying the thickness d, that is, insufficient-thickness portions T1 are present around the hole H1 at portions corresponding to the inclined surfaces of the concave portion 111. It is difficult to detect the insufficient-thickness portions T1 through the analysis of the shapes of the holes in the captured image.

Further, the hole H2 is formed by the second round of cutting on the inner side (a side closer to the bottom of the concave portion 111) than in the first round, and is detected by the hole detection unit 3. The hole H2 detected here is also a region in which there is no log 100 on a path shown by a two-dot chain line and along which the knife 12 passes. Since the hole H2 is formed at a position deeper than in the first round, the hole H2 formed by the second round is smaller than the hole H1 formed by the first round. Insufficient-thickness portions T2 are also present around the hole H2.

Similarly, the hole H3 is formed by the third round of cutting on the inner side (a side closer to the bottom of the concave portion 111) than in the second round, and is detected by the hole detection unit 3. The hole H3 formed by the third round is even smaller than the hole H2 formed by the second round. Insufficient-thickness portions T3 are also present around the hole H3.

In the fourth round of cutting, no hole is detected by the hole detection unit 3 since a region in which there is no log 100 is not present on a path shown by a two-dot chain line and along which the knife 12 passes. However, an insufficient-thickness portion T4 is present at a position corresponding to the bottom of the concave portion 111. Although not shown, no hole is detected by the hole detection unit 3 and no insufficient-thickness portion is present in the fifth round or later.

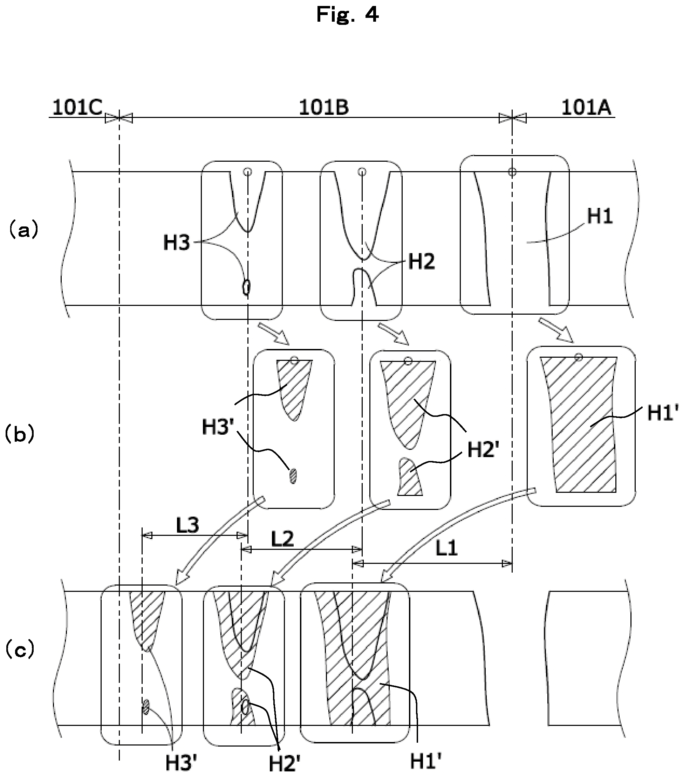

Referring back to FIG. 2, the description will be provided. The veneer length-per-round calculation unit 4 calculates the length of a veneer, which is formed through peeling by one rotation as a veneer length per round in each round of the log 100. In a case where the surface of the log 100 is cut by the knife 12 as in rotary peeling while the log 100 is being rotated, the radius of the log 100 is reduced by the thickness d with each rotation and the length of the veneer 101 formed through peeling per rotation of the log 100 is shortened in each round. The length of the veneer 101 each rotation, which is shortened in each round, that is, each of veneer lengths L1, L2, and L3 per round is shown in FIG. 4 (details will be described later). The veneer length-per-round calculation unit 4 sequentially calculates the veneer lengths L1, L2, and L3 per round each time a hole is detected by the hole detection unit 3 in each round after a hole serving as a starting point (hereinafter referred to as a starting hole) is detected by the hole detection unit 3.

Here, the starting hole is a hole that is detected first in the continuous veneer 101B including unusable portions. In a case where the discontinuous veneer 101A is formed through peeling as shown in FIG. 8 immediately after the cutting of the log 100 is started, a hole crossing in the width direction (hereinafter referred to as a widthwise-crossing hole) is detected by the hole detection unit 3 while the discontinuous veneer 101A is being formed through peeling. When widthwise-crossing holes are successively detected, the widthwise-crossing holes are not regarded as the starting hole. When a hole not crossing in the width direction is detected after the widthwise-crossing hole is detected, the last detected widthwise-crossing hole is regarded as the starting hole. Note that, in a case where the discontinuous veneer 101A is not formed through peeling immediately after the cutting of the log 100 is started and no widthwise-crossing hole is detected, a hole actually detected first by the hole detection unit 3 is regarded as the starting hole.

First, at a point in time when the starting hole is detected by the hole detection unit 3, the veneer length-per-round calculation unit 4 calculates the length L1 of the veneer 101 per rotation from a reference position set at the hole or near the hole. The reference position is a position that serves as a reference when the veneer lengths L1, L2, and L3 per round are calculated, and may be arbitrarily set. In the example shown in FIG. 4, the center position of the starting hole H1 in the transfer direction is set as the reference position (indicated by a straight two-dot chain line extending in a vertical direction) and the veneer length L1 per round is calculated from the reference position. The center position in the transfer direction can be defined as, for example, a center position from an upstream end portion of the hole in the transfer direction to a downstream end portion thereof in the transfer direction, at the center position of the veneer 101 in the width direction.

Further, in a case where the hole H2 is detected by the hole detection unit 3 at a point in time after one rotation as shown in FIG. 4, the veneer length-per-round calculation unit 4 sets the center position of the hole H2 in the transfer direction as the reference position and calculates the next veneer length L2 per round from the reference position. Furthermore, in a case where the hole H3 is detected by the hole detection unit 3 at a point in time after one rotation, the veneer length-per-round calculation unit 4 sets the center position of the hole H3 in the transfer direction as the reference position and calculates the next veneer length L3 per round from the reference position. In addition, since no hole is detected by the hole detection unit 3 at a point in time after another rotation, the veneer length-per-round calculation unit 4 does not calculate the next veneer length per round.

Note that the reference positions for veneer lengths L1, L2, and L3 per round may be most downstream end portions of the holes H1, H2, and H3 in the transfer direction or positions away from the most downstream end portions to the downstream side in the transfer direction by predetermined distances, most upstream end portions of the holes H1, H2, and H3 in the transfer direction or positions away from the most upstream end portions to the upstream side in the transfer direction by predetermined distances, or the like.

Specific processing contents of the veneer length-per-round calculation unit 4 will be described as follow. The knife position detection unit 4A detects the position of the knife 12, which is moved in a direction toward the spindle center 11 with the progress of the cutting of the log 100 performed by the veneer lathe 10, based on the information on the horizontal movement distance of the knife 12 acquired by the knife movement distance acquisition unit 2. The position of the knife 12 means a position to which the knife 12 has been horizontally moved in a direction toward the spindle center 11 from an initial position at the time of start of the cutting of the log 100 (hereinafter referred to as a knife position).

For example, the knife position detection unit 4A sequentially detects the knife position based on the information on the horizontal movement distance that is sequentially acquired from the knife movement distance sensor 13 by the knife movement distance acquisition unit 2. Further, the knife position detection unit 4A responds to the transfer distance calculation unit 4B with a knife position, which is detected at the time of acquisition of a request, in response to a request from the transfer distance calculation unit 4B. Alternatively, the knife position detection unit 4A may transmit an information acquisition request to the knife movement distance sensor 13 in response to a request from the transfer distance calculation unit 4B when a starting hole is detected from the continuous veneer 101B by the hole detection unit 3; may detect the knife position based on the information on the horizontal movement distance of the knife 12 that is acquired as a response thereto; and may respond to the transfer distance calculation unit 4B.

The transfer distance calculation unit 4B calculates a veneer transfer distance between the knife position detected by the knife position detection unit 4A and the position of the line sensor camera 33 (corresponding to an imaging device for acquiring information provided for the detection of a hole performed by the hole detection unit 3). The position of the line sensor camera 33 is a position where the position of the line sensor camera 33 is perpendicularly projected onto the veneer transfer conveyor 21 (hereinafter referred to as a sensor position), and is known information. The knife position, which is used for the calculation of the transfer distance calculation unit 4B, is the position of the knife 12 when the starting hole is detected by the hole detection unit 3. Accordingly, the veneer transfer distance means a distance on the veneer transfer conveyor 21 between the starting hole and the knife 12 at a point in time when the starting hole is detected by the hole detection unit 3.

Note that, in a case where the initial position of the knife 12 at the time of start of the cutting of the log 100 is predetermined (for example, a home position or the like of the knife 12), the veneer transfer distance can be calculated from the sum of the horizontal movement distance of the knife 12 acquired by the knife movement distance acquisition unit 2 and a known separation distance between the home position of the knife 12 and the line sensor camera 33. In this case, the knife position detection unit 4A only needs to have a function of detecting the information on the horizontal movement distance, which is acquired from the knife movement distance acquisition unit 2, as the knife position.

The log radius acquisition unit 4C back-calculates a knife position, which is obtained when the starting hole detected by the hole detection unit 3 is formed through the cutting performed by the knife 12, based on the veneer transfer distance that is calculated by the transfer distance calculation unit 4B when the starting hole is detected by the hole detection unit 3; and acquires a distance between the back-calculated knife position and the spindle center 11 as in-cutting log radius information.

For example, the log radius acquisition unit 4C calculates a transfer time, which is required to transfer the veneer 101 by the veneer transfer distance calculated by the transfer distance calculation unit 4B, based on a transfer distance per unit time of the veneer 101 that is transferred by the veneer transfer conveyors 21. Next, the log radius acquisition unit 4C calculates a movement distance, by which the knife 12 is moved during the transfer time, based on the transfer time and a movement distance of the knife 12 per unit time, and back-calculates a knife position that is horizontally moved to a side opposite to the spindle center 11 by the movement distance. Then, the log radius acquisition unit 4C acquires a distance between the back-calculated knife position and the spindle center 11 as in-cutting log radius information (the radius of the log 100 when the starting hole detected by the hole detection unit 3 at the position of the line sensor camera 33 is present at the position of the knife 12).

The veneer length calculation unit 4D sequentially calculates veneer lengths L1, L2, ... per round in each round of the log 100 each time a hole is detected by the hole detection unit 3 in each round, based on the in-cutting log radius information acquired by the log radius acquisition unit 4C and the thickness information of the veneer 101 that is known information. The thickness information is information preset depending on the veneer 101 to be formed through peeling, and is information indicated by the thickness d in the present embodiment.

Here, in a case where the in-cutting log radius acquired by the log radius acquisition unit 4C is denoted by r1, the veneer length L1 per round in the first round can be calculated based on the in-cutting log radius r1. In a case where the radius of the log 100 at a point in time when the log 100 has completed one rotation is denoted by r2 (=r1-d), the veneer length L2 per round in the second round can be calculated based on this log radius r2. Similarly, the veneer length L3 per round in the third round can be calculated based on a log radius r3 (=r2-d). Note that, each time the holes H1, H2, or H3 is detected by the hole detection unit 3, the processing of the knife position detection unit 4A, the transfer distance calculation unit 4B, and the log radius acquisition unit 4C may be performed to calculate the in-cutting log radius r1, r2, or r3 in each round.

The insufficient-thickness estimation unit 5 aligns the shape of a hole, which is obtained in a specific round and detected by the hole detection unit 3, with a position after one rotation that is shifted to the upstream side in the transfer direction by the veneer length per round calculated by the veneer length-per-round calculation unit 4, and estimates a region, which is defined by the aligned shape of the hole obtained in the specific round, as a potential insufficient-thickness region. That is, an insufficient-thickness portion is estimated to be included in the region defined by the aligned shape of the hole obtained in the specific round. Here, the specific round refers to each round during a period in which a hole is being detected in the continuous veneer 101B, and includes the first round, the second round, the third round, and the like. In contrast, since a potential insufficient-thickness region is estimated after one rotation from a specific round, a round in which the potential insufficient-thickness region is estimated includes the second round, the third round, the fourth round, and the like.

FIG. 4 is a schematic diagram illustrating the processing contents of the insufficient-thickness estimation unit 5. In FIG. 4(a) on the uppermost stage is a diagram showing the veneer 101 (the discontinuous veneer 101A, the continuous veneer 101B including unusable portions, and the continuous veneer 101C including no unusable portion) formed through peeling by the veneer lathe 10. The right side in FIG. 4(a) is the downstream side in the transfer direction, and the left side in FIG. 4(a) is the upstream side in the transfer direction. In FIG. 4(a), the center positions of holes H1, H2, and H3, which are sequentially detected from the continuous veneer 101B by the hole detection unit 3, in the transfer direction are set as reference positions, and veneer lengths L1, L2, and L3 per round in each round of the log 100 are shown on the downstream sides of the reference positions, respectively.

In FIG. 4(b) on the middle stage shows the shapes of the holes H1, H2, and H3 detected from the veneer 101B by the hole detection unit 3 (hereinafter referred to as hole shapes H1', H2', and H3'). In FIG. 4(c) on the lowermost stage shows a state in which the hole shapes H1', H2', and H3' detected by the hole detection unit 3 are aligned with positions after one rotation in the veneer 101 shown in FIG. 4(a), respectively. That is, the hole shape H1' detected in the first round is aligned with a position in the second round that is shifted to the upstream side by the veneer length L1 per round, the hole shape H2' detected in the second round is aligned with a position in the third round that is shifted to the upstream side by the veneer length L2 per round, and the hole shape H3' detected in the third round is aligned with a position in the fourth round that is shifted to the upstream side by the veneer length L3 per round.

The insufficient-thickness estimation unit 5 estimates regions defined by the hole shapes H1', H2', and H3' shown in FIG. 4(c) (regions indicated by hatching) as potential insufficient-thickness regions. That is, the insufficient-thickness estimation unit 5 estimates a region that is defined by the hole shape H1' detected in the first round at a position in the second round with which the hole shape H1' detected in the first round is aligned based on the veneer length L1 per round, as the potential insufficient-thickness region of the second round.

Similarly, the insufficient-thickness estimation unit 5 estimates a region that is defined by the hole shape H2' detected in the second round at a position in the third round with which the hole shape H2' detected in the second round is aligned based on the veneer length L2 per round, as the potential insufficient-thickness region of the third round.

Similarly, the insufficient-thickness estimation unit 5 estimates a region that is defined by the hole shape H3' detected in the third round at a position in the fourth round with which the hole shape H3' detected in the third round is aligned based on the veneer length L3 per round, as the potential insufficient-thickness region of the fourth round.

Note that a state in which the hole shapes H1', H2', and H3' detected by the hole detection unit 3 are aligned with the veneer 101 as in FIG. 4(c) is shown here, but this is for convenience of description and the hole shapes H1', H2', and H3' do not necessarily need to be aligned with the veneer 101. That is, the hole shapes {H1', H2', and H3'} in specific rounds only need to be aligned with the hole shapes {H2', H3', no hole} after one rotation, respectively.

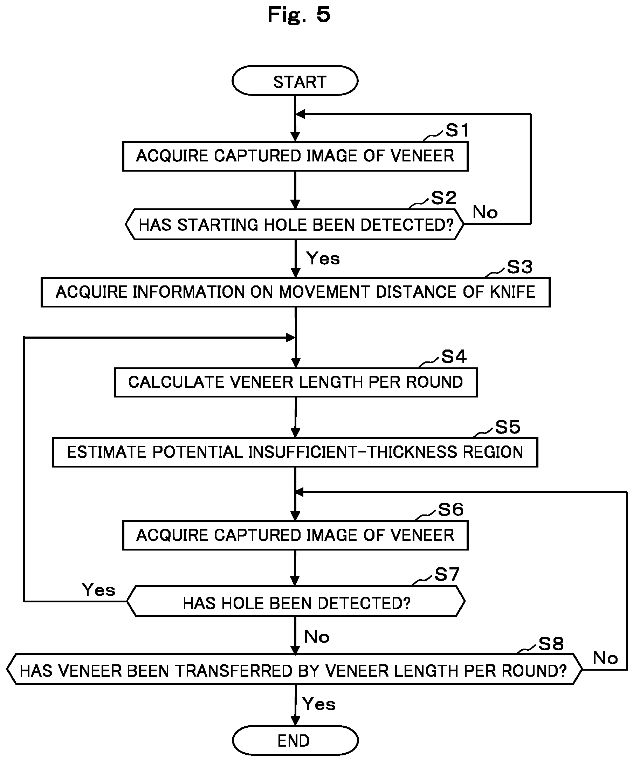

FIG. 5 is a flowchart showing an operation example of the defect determination device 31 configured as described above. The processing of the defect determination device 31 shown in the flowchart of FIG. 5 is started, for example, simultaneously with the start of the operation of the processing line for logs 100.

First, the image acquisition unit 1 sequentially acquires single-line captured images that are generated every predetermined sampling time by the line sensor camera 33, and sequentially combines the respective acquired single-line captured images (Step S1). The hole detection unit 3 determines whether or not a starting hole (the first hole H1 shown in FIG. 4) has been detected in the continuous veneer 101B by analyzing the captured image of the veneer 101 that is acquired by the image acquisition unit 1 (Step S2). Here, in a case where it is determined that the first hole H1 of the continuous veneer 101B has not been detected, the processing returns to Step S1 and the acquisition of the captured image of the veneer 101 is continued.

On the other hand, in a case where the hole detection unit 3 determines that the first hole H1 of the continuous veneer 101B has been detected, the veneer length-per-round calculation unit 4 calculates the veneer length L1 per round in the first round while using the center position of the hole H1 in the transfer direction as a reference position by the processing of Steps S3 and S4. That is, the veneer length-per-round calculation unit 4 outputs an information acquisition request to the knife movement distance acquisition unit 2. The knife movement distance acquisition unit 2 acquires information on the horizontal movement distance of the knife 12 detected by the knife movement distance sensor 13 in response to this information acquisition request, and responds to the veneer length-per-round calculation unit 4 (Step S3). The veneer length-per-round calculation unit 4 calculates a veneer transfer distance based on the information on the horizontal movement distance of the knife 12, calculates an in-cutting log radius r1 by back-calculating a knife position based on the veneer transfer distance, and calculates the veneer length L1 per round of the veneer 101 in the first round from the in-cutting log radius r1 and the thickness d of the veneer 101 (Step S4).

Next, the insufficient-thickness estimation unit 5 aligns the shape of the first hole H1, which is detected in Step S2 by the hole detection unit 3, with a position after one rotation that is shifted to the upstream side in the transfer direction by the veneer length L1 per round in the first round, and estimates a region, which is defined by the aligned shape of the hole H1, as a potential insufficient-thickness region (Step S5).

After that, the image acquisition unit 1 sequentially acquires single-line captured images that are generated every predetermined sampling time by the line sensor camera 33, and sequentially combines the respective acquired single-line captured images (Step S6). The hole detection unit 3 determines whether or not the next hole has been detected by analyzing the captured image of the veneer 101 that is acquired by the image acquisition unit 1 (Step S7).

Here, in a case where the hole detection unit 3 determines in Step S7 that the next hole has not been detected, the insufficient-thickness determination unit 5 determines whether or not the transfer distance of the veneer 101 from a point in time when the previous hole is detected by the hole detection unit 3 exceeds the veneer length per round calculated by the veneer length-per-round calculation unit 4 (Step S8). In a case where the transfer distance of the veneer 101 does not exceed the veneer length per round, the processing returns to Step S6 and the acquisition of the captured image of the veneer 101 is continued.

On the other hand, in a case where the hole H2 following the hole H1 is detected in Step S7 by the hole detection unit 3, the processing returns to Step S4 since the continuous veneer 101B is still continued. In Step S4, the veneer length-per-round calculation unit 4 calculates the veneer length L2 per round in the second round using the center position of the hole H2 in the transfer direction as a reference position. Then, in Step S5, the insufficient-thickness estimation unit 5 aligns the shape of the hole H2 with a position that is shifted to the upstream side in the transfer direction by the veneer length L2 per round in the second round, and estimates a region, which is defined by the aligned shape of the hole H2, as a potential insufficient-thickness region.

Hereinafter, in the same manner, when the hole H3 is detected in Step S7, the processing returns to Step S4 and the veneer length L3 per round in the third round is calculated and a potential insufficient-thickness region is estimated with the shape of the hole H3 aligned with a position corresponding to the veneer length L3 per round (Step S5).

In the loop processing of Steps S4 to S8 as described above, in a case where it is determined that the transfer distance of the veneer 101 exceeds the veneer length L3 per round in the third round (Yes in Step S8) in a state in which a hole following the hole H3 is not detected (No in Step S7), the section of the continuous veneer 101B including unusable portions y and usable portions f ends and the section of the continuous veneer 101C including only usable portions f begins. Therefore, the processing of the flowchart shown in FIG. 5 ends. Note that since the continuous veneer 101C is actually transferred thereafter, the processing of the image acquisition unit 1 and the hole detection unit 3 is continuously performed.

As described in detail above, in the present embodiment, the holes H1, H2, and H3 are detected in the continuous veneer 101B that is formed through peeling from the log 100 rotating about the spindle center 11 by the veneer lathe 10 and is transferred on the veneer transfer conveyors 21, and the length of the veneer 101B formed through peeling by one rotation in each round of the log 100 is calculated as the veneer lengths L1, L2, and L3 per round. Then, the shapes of the detected holes H1, H2, and H3 obtained in specific rounds are aligned with positions after one rotation that are shifted to the upstream side in the transfer direction by the veneer lengths L1, L2, and L3 per round, respectively, and regions defined by the aligned shapes of the holes H1, H2, and H3 obtained in the specific rounds are estimated as potential insufficient-thickness regions (regions in which insufficient-thickness portions T2, T3, and T4 may be included).

According to the present embodiment configured as described above, it can be estimated that the insufficient-thickness portions T2, T3, and T4, which are formed as the concave portion 111 having an inclination on the surface layer portion of the log is cut by the knife 12 of the veneer lathe 10, may be included in regions with which the shapes of the holes H1, H2, and H3 detected in the respective rounds of the log 100 are aligned based on the veneer lengths L1, L2, and L3 per round in respective rounds. That is, according to the present embodiment, it is possible to estimate regions that may include the insufficient-thickness portions T2, T3, and T4, which are included in the surface layer portion of the log and cannot be easily determined by a method of determining whether or not an object is present with reflected light using a photoelectric sensor or the like and a method of observing a difference in color of a captured image.

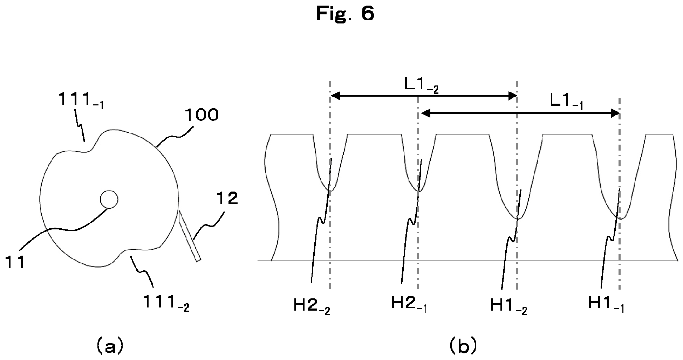

Note that an example in which only one concave portion 111 is present on the surface layer portion of the log 100 as shown in FIG. 3(a) and only one hole is detected during one rotation of the log 100 has been described in the embodiment. In contrast, there is also a case where concave portions 111₋₁ and 111₋₂ are present at a plurality of locations on the surface layer portion of the log 100 and a plurality of holes are detected during one rotation of the log 100 as shown in FIG. 6. In this case, it may also be possible to estimate potential insufficient-thickness regions by calculating veneer lengths {L1₋₁, L2₋₁, ...} and {L1₋₂, L2₋₂, ...} per round while setting each of a plurality of holes H1₋₁ and H1₋₂, which are detected in the continuous veneer 101B in the first one rotation, as a starting hole.

In this case, for example, in a case where the hole H1₋₁ is detected first in the continuous veneer 101B by the hole detection unit 3, the veneer length-per-round calculation unit 4 calculates the veneer length L1₋₁ per round in the first round from the reference position of the hole H1₋₁ to the upstream side in the transfer direction while setting the hole H1₋₁ as a starting hole. Further, in a case where another hole H1₋₂ is detected by the hole detection unit 3 before the veneer 101B is transferred by the veneer length L1₋₁ per round, the veneer length-per-round calculation unit 4 calculates the veneer length L1₋₂ per round in the first round from the reference position of the hole H1₋₂ to the upstream side in the transfer direction while also setting the hole H1₋₂ as a starting hole. Then, veneer lengths {L1₋₁, L2₋₁, ...} and {L1₋₂, L2₋₂, ...} per round in the respective rounds are sequentially calculated for each of the two starting holes H1₋₁ and H1₋₂.

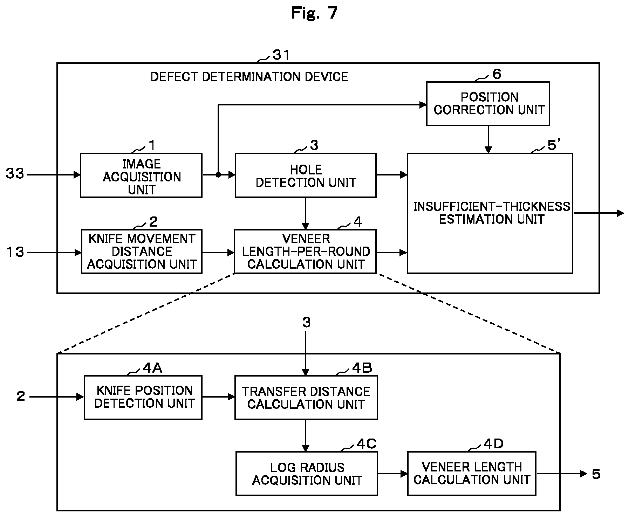

Furthermore, in the embodiment, the position of a hole to be aligned may be corrected using the captured image acquired by the line sensor camera 33 (an imaging device for acquiring information provided for the detection of a hole performed by the hole detection unit 3). For example, as shown in FIG. 7, the defect determination device 31 may further include a position correction unit 6 and may include an insufficient-thickness determination unit 5' instead of the insufficient-thickness determination unit 5.

The veneer 101 cut in the veneer lathe 10 has stretchability due to the occurrence of back checks (lathe checks) during cutting. On the other hand, since the veneer 101 is a natural object, the veneer 101 also includes portions that are less likely to be deformed due to variations in density, knots, or the like. For this reason, the veneer 101 may be irregularly deformed during the transfer of the veneer 101 on the rear side of the veneer lathe 10. As a result, even though a position with which the shape of a hole is to be aligned is determined using the veneer lengths L1, L2, and L3 per round, it may be difficult to perform accurate alignment due to dimensional errors of the deformed veneer 101.

In this regard, the position correction unit 6 performs image matching by comparing a captured image (hereinafter referred to as a specific round-captured image) acquired in a specific round of the log 100 by the line sensor camera 33 and a captured image (hereinafter referred to as the next round-captured image) acquired in a round following the specific round by the line sensor camera 33, and corrects the position of a hole to be aligned, based on a positional deviation between the specific round-captured image and the next round-captured image. The insufficient-thickness determination unit 5' aligns the shape of a hole in the specific round, which is detected by the hole detection unit 3, with a position corrected by the position correction unit 6.

The method of image matching is arbitrary. For example, image feature information can be acquired from each of a specific round-captured image of a predetermined range of the periphery of a hole that is acquired in a specific round by the image acquisition unit 1 and the next round-captured image of the predetermined range that is acquired by the image acquisition unit 1 after one rotation from the specific round (a captured image of the predetermined range including a position shifted to the upstream side in the transfer direction by the veneer length per round calculated by the veneer length-per-round calculation unit 4); and the matching of image patterns can be performed through the comparison of the image feature information. Feature quantities obtained through the Fourier transform of a captured image can be used as an example of the image feature information. Here, since a hole appearing in the specific round-captured image and a hole appearing in the next round-captured image are different from each other in size, it is preferable that matching is performed for the image of a region other than the hole.

In a case where the presence of a positional deviation between the specific round-captured image and the next round-captured image is detected by image matching, the position correction unit 6 corrects a position with which a hole shape detected from the specific round-captured image is to be aligned, based on the amount and direction of the deviation. In the present embodiment, image matching is performed also using the veneer length per round calculated by the veneer length-per-round calculation unit 4. Accordingly, even in a case where a hole having a similar shape is detected at a position different from the position defined by the veneer length per round, it is possible to estimate a potential insufficient-thickness region with high accuracy by performing accurate image matching without being affected by the hole.

An example in which the line sensor camera 33 (an imaging device capable of capturing a color image) is used as a sensor for acquiring information provided for the detection of the hole performed by the hole detection unit 3 has been described in the embodiment, but the present invention is not limited thereto. For example, a plurality of photoelectric sensors arranged on a line in the width direction of the veneer 101 may be used. In this case, the hole detection unit 3 can detect a hole by analyzing information, which is acquired by the plurality of photoelectric sensors, with a simple method not using image processing.

In addition, the embodiments are merely specific examples for carrying out the present invention, and the technical scope of the present invention should not be interpreted in a limited manner by the embodiments. That is, the present invention can be implemented in various forms without departing from the gist or essential features thereof.

### Reference Signs List

- 1:: image acquisition unit
- 2:: knife movement distance acquisition unit
- 3:: hole detection unit
- 4:: veneer length-per-round calculation unit
- 4A:: knife position detection unit
- 4B:: transfer distance calculation unit
- 4C:: log radius acquisition unit
- 4D:: veneer length calculation unit
- 5, 5':: insufficient-thickness estimation unit
- 6:: position correction unit
- 10:: veneer lathe
- 11:: spindle center (center of rotation of log)
- 12:: knife
- 13:: knife movement distance sensor
- 21, 22:: veneer transfer conveyor
- 30:: defect inspection system
- 31:: defect determination device
- 32:: illumination device
- 33:: line sensor camera (imaging device)
- 40:: veneer cutting apparatus
- 41:: cutting control device

## Claims

1. A veneer defect determination device comprising:
a hole detection unit that detects a hole in a veneer formed through peeling from a log rotating about a spindle center by a veneer lathe and transferred on a transfer path;
a veneer length-per-round calculation unit that calculates a length of the veneer formed through peeling by one rotation as a veneer length per round in each round of the log; and
an insufficient-thickness estimation unit that aligns a shape of a hole, which is obtained in a specific round and detected by the hole detection unit, with a position after one rotation, the position after one rotation being shifted to an upstream side in a transfer direction by the veneer length per round calculated by the veneer length-per-round calculation unit, and estimates that an insufficient-thickness portion is included in a region defined by the aligned shape of the hole obtained in the specific round.

2. The veneer defect determination device according to claim 1,
wherein the veneer length-per-round calculation unit calculates the veneer length per round in each round of the log, based on in-cutting log radius information representing a radius of the log when a hole serving as a starting point detected by the hole detection unit is formed through cutting performed by the veneer lathe, and on thickness information of the veneer to be formed through peeling.

3. The veneer defect determination device according to claim 2,
wherein the veneer length-per-round calculation unit includes
a knife position detection unit that detects a position of a knife moving in a direction toward the spindle center with a progress of cutting of the log performed by the veneer lathe,
a transfer distance calculation unit that calculates a veneer transfer distance between a knife position detected by the knife position detection unit and a position of a sensor for acquiring information provided for detection of the hole performed by the hole detection unit,
a log radius acquisition unit that back-calculates a knife position, which is obtained when the hole serving as the starting point is formed through cutting performed by the knife, based on the veneer transfer distance calculated by the transfer distance calculation unit when the hole serving as the starting point is detected by the hole detection unit, and acquires a distance between the back-calculated knife position and the spindle center as the in-cutting log radius information, and
a veneer length calculation unit that calculates the veneer length per round in each round of the log, based on the in-cutting log radius information acquired by the log radius acquisition unit and the thickness information of the veneer.

4. The veneer defect determination device according to claim 2,
wherein, in a case where another hole is detected by the hole detection unit in a section of a veneer length per round in a first round calculated on an upstream side in a transfer direction from a position of a hole detected first by the hole detection unit, the veneer length-per-round calculation unit calculates the veneer length per round in each round of the log while each of a plurality of holes including the hole detected first and the other hole is set as the hole serving as the starting point.

5. The veneer defect determination device according to any one of claims 1 to 4,
wherein the sensor for acquiring information provided for detection of the hole performed by the hole detection unit is an imaging device that captures an image of the veneer, and
the hole detection unit detects the hole by analyzing the captured image acquired by the imaging device.

6. The veneer defect determination device according to claim 5, further comprising:
a position correction unit that performs image matching by comparing a specific round-captured image acquired in the specific round by the imaging device and a next round-captured image acquired in a round following the specific round by the imaging device, and corrects a position of a hole to be aligned, based on a positional deviation between the specific round-captured image and the next round-captured image.

7. The veneer defect determination device according to any one of claims 1 to 4,
wherein the sensor for acquiring information provided for detection of the hole performed by the hole detection unit is a plurality of photoelectric sensors arranged on a line in a width direction of the veneer, and
the hole detection unit detects the hole by analyzing information acquired by the plurality of photoelectric sensors.

8. A veneer defect determination method comprising:
a step of detecting a hole in a veneer formed through peeling from a log rotating about a spindle center by a veneer lathe and transferred on a transfer path by a hole detection unit of a defect determination device;
a step of calculating a length of the veneer formed through peeling by one rotation as a veneer length per round in each round of the log by a veneer length-per-round calculation unit of the defect determination device; and
a step of aligning a shape of a hole, which is obtained in a specific round and detected by the hole detection unit, with a position after one rotation by an insufficient-thickness estimation unit of the defect determination device, the position after one rotation being shifted to an upstream side in a transfer direction by the veneer length per round calculated by the veneer length-per-round calculation unit, and of estimating that an insufficient-thickness portion is included in a region defined by the aligned shape of the hole obtained in the specific round by the insufficient-thickness estimation unit.

9. A veneer defect determination program for causing a computer to function as:
hole detection means for detecting a hole in a veneer formed through peeling from a log rotating about a spindle center by a veneer lathe and transferred on a transfer path;
veneer length-per-round calculation means for calculating a length of the veneer formed through peeling by one rotation as a veneer length per round in each round of the log; and
insufficient-thickness estimation means for aligning a shape of a hole, which is obtained in a specific round and detected by the hole detection means, with a position after one rotation, the position after one rotation being shifted to an upstream side in a transfer direction by the veneer length per round calculated by the veneer length-per-round calculation means, and for estimating that an insufficient-thickness portion is included in a region defined by the aligned shape of the hole obtained in the specific round.
